# EUROPEAN PATENT SPECIFICATION

(11) **EP 1 231 928 B1**
(45) Date of publication and mention of the grant of the patent: **18.06.2003**
(21) Application number: 00980105.1
(22) Date of filing: 03.11.2000
(51) Int. Cl.: A61K 35/78, A61P 37/00

(54) **PHYTOTHERAPEUTIC COMPOSITION**
PHYTOTHERAPEUTISCHE ZUBEREITUNG
COMPOSITION PHYTOTHERAPEUTIQUE

(30) Priority: 04.11.1999 NL 1013497
(43) Date of publication of application: 21.08.2002
(73) Proprietor: Brandes, Maria, Antoinette, 4725 SR Wouwse Plantage (NL); Brandes, Jacobus, Marinus, 4725 SR Wouwse Plantage (NL)
(72) Inventor: Brandes, Maria, Antoinette, 4725 SR Wouwse Plantage (NL); Brandes, Jacobus, Marinus, 4725 SR Wouwse Plantage (NL)
(74) Representative: Verhees, Godefridus Josephus Maria
(86) International application number: NL0000805
(87) International publication number: WO01034170

(56) References cited:
- WO-A-98/42188
- DATABASE BIOSIS [Online] BIOSCIENCES INFORMATION SERVICE, PHILADELPHIA, PA, US; March 1998 (1998-03) GRELA EUGENIUSZ R ET AL: "Immunostimulatory activity of herbs." Database accession no. PREV199800184730 XP002169406 & MEDYCYNA WETERYNARYJNA, vol. 54, no. 3, March 1998 (1998-03), pages 152-158, ISSN: 0025-8628
- DATABASE FSTA [Online] INTERNATIONAL FOOD INFORMATION SERVICE (IFIS), FRANFURT/MAIN, DE; SHUN-JEN TSAI: "Physiological effects of bioactive components of Allium species." Database accession no. 1998-05-j0981 XP002169407 & FOOD SCIENCE, TAIWAN 1997 DEP. OF FOOD SCI., NAT. CHUNG-HSING UNIV., TAICHUNG, TAIWAN, vol. 24, no. 6, pages 629-648,

## Description

### BACKGROUND OF THE INVENTION:

### Field of the invention

The invention relates to a phytotherapeutic composition suitable for the stimulation of the immune system of humans and animals and for improving the condition of the immune system of a human or animal.

### Prior art

It is known of many herbs that they have a positive effect on the immune system of living creatures. The active ingredients in the herbs are often obtained by extraction from the herbs. The extracts are often administered dissolved in or mixed with water via oral ingestion. From WO-A 98/42188 a composition is known for use in treating diseases or helping prevent the transmission thereof comprising among others Echinacea purpurea and allium.

### Summary of the invention

An objective of the invention is to provide alternative phytotherapeutic compositions for the stimulation of the immune system in a human or animal and the improvement of the condition of the immune system of a human or animal. To this end the phytotherapeutic composition according to the invention is characterised in that the composition contains extracts of Allium sativum, Juglans regia, Echinacea purpurea and Viola tricolor. This composition improves the general condition of humans or animals, it improves resistance to stress and pathogens, and it encourages recovery from illness.

The improvement of the general condition can be explained as follows. The general condition is closely related to the direct living environment of cells, the interior environment. In essence, the composition of this depends on 3 factors: the supply of nutrients and fuel (digestion), the elimination and discharge of waste products (liver and kidneys), and the metabolic activity in the cells themselves. A chronic contamination of the interior environment leads to such situations as a disrupted energy balance, which encourages weight loss and susceptibility to disease.

Allium sativum has a regulatory effect on the bowels. Garlic preparations have a weak spasmolytic effect on cramped bowel musculature and fight meteorism. Minimal quantities further digestion, increase bowel tone and peristalsis. Allium is a stomachic and stimulates gastrointestinal gland secretion. Improved bowel motility and improvement in the secretion of digestive juices furthers the absorption of nutrients from food, which in addition to growth also provides extra energy in order to fight possible infections and repair damaged tissue.

A well-balanced bowel flora system is crucial for optimal digestion. Garlic has a regulatory effect on bowel commensals. A possible imbalance due to the excessive development of a certain type of bacteria can be effectively corrected by Allium sativum.

Allium sativum also has a cleansing and stimulating effect on blood circulation, so that absorbed nutrients can rapidly penetrate into the interior environment. Garlic lowers the cholesterol level in the blood, has vasospasmolytic characteristics and furthers the coronary circulation of the myocardium.

Small molecular metabolic waste is often removed from the body by the urinary system. Viola tricolor contains much of the flavonoid Rutin that furthers diuresis. The plant is known in popular medicine as a haematic that can be useful in various illnesses with a metabolic component (exudative diathesis, rheumatism, gout, arteriosclerosis, etc.) and in skin conditions (exanthema, eczema, acne, pyodermia, etc.).

By influencing both the liver and the kidneys the phytotherapeutic composition according to the invention is able to stimulate the purification of the interior environment on a broad level.

The favourable effect on the increase of the resistance capacity against infections is expressed in improved inflammation inhibition and increased immunity.

The inflammation inhibiting property can be explained as follows. Juglon (5-oxy 1,4 naphthochinone) is chemically strongly related to the antibiotic naphthochinone derivate Plumbagine (2-methyl 5-oxy-1,4-naphthochinone.) Plumbagine is a bactericide especially for strepto-, pneumo-, and staphylococci. In recent studies it was shown in vitro that an alcohol extract of Juglans regia had a bactericidal effect on Staphylococcus aureus and Staphylococcus enteriditis, but not on Escherichia coli.

Echinacea makes it more difficult for the pathogenic microorganism to infect tissue and to spread. Echinacea inhibits the hyalurone production of connective tissue, such that microorganisms can less easily adhere to connective tissue cells. In addition, the tincture extract (caffeic acid conjugates) shows bactericidal characteristics against various bacterial pathogens (including coli and pseudomonas strains, Staphylococcus and E. coli).

The increase in immunity can be explained as follows. Juglans regia is an herb that contains many gallo- and catecholtannins and naphthochinones. In popular medicine extracts of the leaf are highly appreciated for their purifying effect on the blood and lymphatic system. Juglans regia is often used for gastrointestinal infections with diarrhoea symptoms and for mild surface skin infections. Although the clinical applications have not yet been sufficiently scientifically documented, a favourable effect on diarrhoea and surface damage by infection seems plausible. Juglans regia has a high content of tannins and naphthochinone derivatives. Tannins have a strong astringent effect on irritated surfaces (mucosa or skin) and the disinfecting characteristics of naphthochinone derivatives hydrojuglon and juglon can favourably influence the combating of infections.

Echinacea is a widely known prophylactic against both viral and bacterial infections. Tincture extract increases cellular and humoral resistance, reinforces leucocytic mobilisation, stimulates macrophages and increases the discharge of interleukins. The plant contains caffeic acid derivatives, polysaccharides and glycoproteins that stimulate phagocytosis and NK cell activity. In addition, there are also mild analgesic alkylamides that inhibit the infectious process. Echinacea also stimulates the activity of strain cells, B- and T-cells. Just like Eleutherococcus, Echinacea stimulates, via ACTH, the corticosteroid production of the adrenal glands. Corticosteroids stimulate the activity of fully-grown lymphocytes. So it is probable that both herbs, in a phytotherapeutic composition according to the invention, demonstrate synergy by stimulating the immune system in a hormonally controlled stress reaction. Echinacea does not appear able to stimulate the formation of memory cells. So it appears that acute infectious processes are favourably influenced but that the immune system is not helped to better arm itself against recurrences.

Caffeic acid conjugates, including cichoric acids, appear to have an interferon-like action without, incidentally, being able to stimulate interferon production itself. This reinforcement of viral resistance corresponds with clinical observations that Echinacea makes a clearly positive contribution to recovery from influenza and colds.

Recovery after illness can be explained as follows. As a consequence of infections usually there is damage to tissue and lymph/blood circulation. Due to a defective discharge of waste products and lysed cell material a hypertonic interior environment can arise, which encourages oedema. This can be a negative vicious circle. The pile of waste products attracts fluids, such that internal tissue pressure increases. Thus the peripheral resistance of capillaries increases, which once again inhibits the discharge of waste products. A first condition for tissue recovery, therefore, is to break this vicious circle. The phytotherapeutic composition according to the invention contains two herbs that fight oedema: Echinacea and Viola tricolor.

Echinacea accelerates the recovery of connective tissue and stimulates lymph circulation. Polysaccharides from an alcohol extract appear to increase the activity of fibroblasts and fibrocytes in connective tissue and in the stratum germinativum of the epidermis. In addition, caffeic acid conjugates and alkylamide phagocytes are encouraged on the spot to clean up cellular waste.

Viola tricolor contains many saponins (dissolve mucus, airway infections) and the flavonglycoside Rutin that furthers diuresis. In addition, the leaf has the heteroside Gaultherine that, under the influence of a fermentation process, is dissolved such that methylsalicate is released. Thus, together with the alkylamides of Echinacea, probably modest pain decrease can be obtained. So one can move more easily, which encourages the muscular pumping function to discharge more moisture.

Studies have shown that the phytotherapeutic composition when administered leads to good results if the ratio of the quantity of the extracts Allium sativum, Juglans regia, Echinacea purpurea and Viola tricolor is approximately 3:2:3:3.

It is noted that from DATABASE BIOSIS [Online] BIOSCIENCES INFORMATION SERVICE, PHILADELPHIA, PA, US; March 1998 (1998-03) GRELA EUGENIUSZ R ET AL: "Immunostimulatory activity of herbs.' Database accession no. PREV199800184730 XP002169406 & MEDYCYNA WETERYNARY JNA, vol. 54, no. 3, March 1998 (1998-03), pages 152-158, ISSN: 0025-8628 a medical composition is known comprising among others Echinacea purpurea, Allium sativum and Eleutherococcus senticus for improving cellular immunity, humoral immunity and having antibacterial and antiviral activity. No Juglans regia or Viola tricolor is mentioned in this publication.

Further it is noted that in DATABASE FSTA [Online] INTERNATIONAL FOOD INFORMATION SERVICE (IFIS), FRANKFURT/MAIN, DE; SHUN-JENTSAI: "Physiological effects of bioactive components of Allium species.' Database accession no. 1998-05-j0981 XP002169407 & FOOD SCIENCE, TAIWAN 1997 DEP. OF FOOD SCI., NAT. CHUNG-HSING UNIV., TAICHUNG, TAIWAN, vol. 24, no. 6, pages 629-648 beneficial effects of garlic on immune system modulation are described.

And it is noted that in European patent application EP 0 225 496 it is described that a pharmaceutical composition can be obtained by isolating polysaccharide particles from Echinacea purpurea by means of extraction. These active ingredients can for example, mixed with water, be administered to animals by oral ingestion. The effect of the administration of these substances is that the immune system of animals is stimulated.

An embodiment of the phytotherapeutic composition according to the invention is characterised in that the composition also contains an extract of Carduus marianus. By adding Carduus marianus the general condition of humans or animals is further improved by the purification of their interior environment.

In the phytotherapeutic composition according to the invention, attention is paid to purifying the interior environment via the blood and lymph. During an infectious process cellular and metabolic waste is produced (including toxins), which can deregulate the immune system. The liver does most of the processing of metabolic waste. The protection and stimulation of the detoxifying liver function during long-term stress and stubborn infections is essential in order to maintain an optimal internal environment. Carduus marianus contains Silymarin, an anti-hepatotoxicant that probably stabilises cell membranes, so that toxins can cause no internal poisoning of hepatocytes. In addition, Silymarin stimulates the action of polymerase A, such that more ribosomal protein is produced to repair internal damage and make new hepatocytes. Silymarin proves to also support the bowel and liver in making free radicals harmless (anti-oxidant effect). It has been demonstrated that Silymarin binds to hydroxyl groups and acid groups of free radical compounds such that the harmful perioxidative effects of these substances decrease. Chemiluminescence studies have indicated that Silymarin also has infection-inhibiting characteristics. In Kupffer cells of the rat there was noted to be an inhibition of the production of leucotrines.

Here, too, it was noted from studies that the phytotherapeutic composition when administered leads to good results if the ratio of the quantity of the extracts Allium sativum, Juglans regia, Echinacea purpurea, Viola tricolor, and Carduus marianus is approximately 3:2:3:3:2.

A further embodiment of the phytotherapeutic composition according to the invention is characterised in that the composition also contains an extract of Uncaria tomentosa. Adding Uncaria tomentosa increases immunity and therefore resistance to infections.

Uncaria tomentosa stimulates and protects on a broad level. The climbing plant contains many compounds including alkaloids, glycosides and phenols. These substances are viewed as central elements when it comes to immune stimulation, inflammation inhibition and cell protection. Glycosides offer protection against viral infections, alkaloids are immune stimulating, and phenols have been associated with antioxidant activity. Particularly oxindole alkaloids stimulate leucocytes and macrophages in response to pathogens. In addition, there are indications that humoral immune response, too (NK cells and antibody production) can be affected by Uncaria extract by influencing the T helper cells.

Juglans regia, Echinacea purpurea and Uncaria tomentosa together form the core of the phytotherapeutic composition according to the invention when it comes to protection against infections. Here Juglans particularly furthers resistance, via the lymphatic system, at an internal (airways, bowel system) and external (skin) level. Echinacea helps resistance, in a broad sense, against bacteria, viruses and moulds/yeasts, while Uncaria particularly reinforces the viral component of resistance and makes cells stronger so that they can survive harmful effects.

The phytotherapeutic composition according to the invention reinforces resistance to infections with three herbs: Juglans regia, Uncaria tomentosa, and Echinacea purpurea. All three combine antiphlogistic characteristics with immune stimulation. Uncaria and Echinacea have a broad influence on the immune system, while Juglans particularly reinforces the lymphatic portion.

Here, once again, studies have shown that the phytotherapeutic composition when administered leads to good results if the ratio of the quantity of the extracts Allium sativum, Juglans regia, Echinacea purpurea, Viola tricolor, Uncaria tomentosa, and Carduus marianus is approximately 3:2:3:4:3:2.

A further embodiment of the phytotherapeutic composition according to the invention is characterised in that the composition also comprises an extract of Eleutherococcus senticosus. By adding Eleutherococcus senticosus (Siberian ginseng), the general condition is improved by improving the energy balance. The herb has a stimulating and vitalising effect because it releases extra energy-rich compounds in the body. Glycosides, also known as eleutherosides, stimulate carbohydrate metabolism in the liver to produce energy-rich phosphate compounds as ATP (and probably GTP as well). In addition, Eleutherococcus raises the blood sugar level. Cells can absorb this extra supply of glucose well, because the plant also furthers the production of the hormones ACTH (adrenocorticotrophic hormone) and insulin. The result of all of this is that more energy is supplied for physical effort, fighting infections and tissue regeneration during recovery from illness, both directly (via ATP/GTP) and indirectly (via glucose).

Eleutherococcus also increases tolerance to stress and general resistance against toxins of a physical, chemical, or biological nature. Particularly the substance Eleutheroside E appears to have a broad regulatory effect that both affects the cells and the neuro-vegetative regulatory mechanisms of the interior environment. Eleutherococcus normalises blood pressure and regulates glucose levels in the blood. On a cerebral level regular use leads to a general feeling of well being to even mild euphoria. The herb does not lead to dependence or exhaustion. So Eleutherococcus stimulates catabolic functions on a broad level and increases mental and physical resistance without any situation of overuse.

There are indications that Eleutherococcus has an effect on the hypothalamohypophysary system. In addition to a favourable effect on the alarm system of the adrenal glands, there is also a regulatory effect perceptible on the function of the sexual organs.

Finally, studies have shown here, as well, that the phytotherapeutic composition when administered leads to good result if the ratio of the quantity of the extracts Allium sativum, Eleutherococcus senticosus, Juglans regia, Echinacea purpurea, Viola tricolor, Uncaria tomentosa and Carduus marianus is approximately 3:3:2:3:4:3:2.

Preferably the phytotherapeutic composition according to the invention comprises 1-30 volume% of Allium sativum extract, 1-30 volume% Eleutherococcus senticosus extract, 1-25 volume% Juglans regia extract, 1-30 volume% Echinacea purpurea extract, 5-35 volume% Violatricolor extract, 1-30 volume% Uncaria tomentosa extract, and 1-25 volume% Carduus marianus extract.

Studies have shown that a composition of at least nearly 15 volume% Allium sativum extract, 15 volume% Eleutherococcus senticosus extract, 10 volume% Juglans regia extract, 15 volume% Echinacea purpurea extract, 20 volume% Viola tricolor extract, 15 volume% Uncaria tomentosa extract and 10 volume% Carduus marianus extract leads to good results.

### Detailed description of the invention

The most extensive embodiment of the phytotherapeutic composition according to the invention comprises a combination of extracts of the herbs Allium sativum or garlic, Eleutherococcus senticosus or Siberian ginseng, Juglans regia or walnut, Echinacea purpurea or purple coneflower, Viola tricolor or violet, Uncaria tomentosa or Cat's claw and Carduus marianus or milk thistle, for example in the ratio of 3:3:2:3:4:3:2. The extracts are obtained in a manner known to experts by extracting the herbs, for example on a water or alcohol basis. Preferably the phytotherapeutic composition according to the invention is comprised of alcohol extracts of the above-mentioned herbs with an alcohol content of approximately 40%.

The phytotherapeutic composition has a broad area of use as a health product. The product furthers the general feeling of well being, increases resistance to stress, stimulates specific and aspecific immunity and harmonises vegetative functioning.

In a prophylactic context it is recommended that the phytotherapeutic composition according to the invention be used when there is still some capacity left for recovery. This means administration before an infection has taken place or during the preliminary phase of an infectious process.

The phytotherapeutic composition accelerates recovery after illness by furthering the recovery of connective tissue and fighting oedema.

In addition to the above-mentioned herbs the phytotherapeutic composition can also contain other components, such as for example scents and flavours if the medication is administered orally.

Although in the above the invention is explained on the basis of the description of one embodiment, it should be noted that the invention is in no way limited to this embodiment. The invention also extends to all embodiments deviating from this embodiment within the context defined by the claims. For example extraction can also take place on a watery acid basis. In addition, the use of the phytotherapeutic composition is not limited to humans and animals. The physiotherapeutic composition can also be used for plants. It has been noted that when administered to plants it furthers their growth and their resistance to disease.

## Claims

1. Phytotherapeutic composition comprising extracts of Allium sativum, Juglans regia, Echinacea purpurea and Viola tricolor.

2. Phytotherapeutic composition according to claim 1, **characterised in that** the ratio of the quantity of the extracts Allium sativum, Juglans regia, Echinacea purpurea and Viola tricolor is approximately 3:2:3:3.

3. Phytotherapeutic composition according to claims 1 or 2, **characterised in that** the composition also contains an extract of Carduus marianus.

4. Phytotherapeutic composition according to claim 3, **characterised in that** the ratio of the quantity of the extracts Allium sativum, Juglans regia, Echinacea purpurea, Viola tricolor, and Carduus marianus is approximately 3:2:3:3:2.

5. Phytotherapeutic composition according to one of the preceding claims, **characterised in that** the composition also contains an extract of Uncaria tomentosa.

6. Phytotherapeutic composition according to claims 1, 3, and 5, **characterised in that** the ratio of the quantity of the extracts Allium sativum, Juglans regia, Echinacea purpurea, Viola tricolor, Uncaria tomentosa, and Carduus marianus is approximately 3:2:3:4:3:2.

7. Phytotherapeutic composition according to one of the preceding claims, **characterised in that** the composition also contains an extract of Eleutherococcus senticosus.

8. Phytotherapeutic composition according to claims 1, 3, 5, and 7, **characterised in that** the ratio of the quantity of the extracts Allium sativum, Eleutherococcus senticosus, Juglans regia, Echinacea purpurea, Viola tricolor, Uncaria tomentosa, and Carduus marianus is approximately 3:3:2:3:4:3:2.

9. Phytotherapeutic composition according to claims 1, 3, 5 and 7 or according to claim 8, **characterised in that** the composition comprises 1-30 volume% Allium sativum extract, 1-30 volume% Eleutherococcus senticosus extract, 1-25 volume% Juglans regia extract, 1-30 volume% Echinacea purpurea extract, 5-35 volume% Viola tricolor extract, 1-30 volume% Uncaria tomentosa extract, and 1-25 volume% Carduus marianus extract.

10. Phytotherapeutic composition according to claim 9, **characterised in that** the composition at least nearly comprises 15 volume% Allium sativum extract, 15 volume% Eleutherococcus senticosus extract, 10 volume% Juglans regia extract, 15 volume% Echinacea purpurea extract, 20 volume% Viola tricolor extract, 15 volume% Uncaria tomentosa extract, and 10 volume% Carduus marianus extract.

## Patentansprüche

1. Phytotherapeutische Zusammenstellung mit Extrakten von Allium sativum, Juglans regia, Echinacea purpurea und Viola tricolor.

2. Phytotherapeutische Zusammenstellung nach Anspruch 1, **dadurch gekennzeichnet, dass** das Mengenverhältnis der Extrakte von Allium sativum, Juglans regia, Echinacea purpurea und Viola tricolor ungefähr 3:2:3:3 beträgt.

3. Phytotherapeutische Zusammenstellung nach Anspruch 1 oder 2, **dadurch gekennzeichnet, dass** die Zusammenstellung darüber hinaus ein Extrakt von Carduus marianus enthält.

4. Phytotherapeutische Zusammenstellung nach Anspruch 3, **dadurch gekennzeichnet, dass** das Mengenverhältnis der Extrakte von Allium sativum, Juglans regia, Echinacea purpurea, Viola tricolor und Carduus marianus ungefähr 3:2:3:3:2 beträgt.

5. Phytotherapeutische Zusammenstellung nach einem der vorangegangenen Ansprüche, **dadurch gekennzeichnet, dass** die Zusammenstellung darüber hinaus ein Extrakt von Uncaria tomentosa enthält.

6. Phytotherapeutische Zusammenstellung nach Anspruch 1, 3 und 5, **dadurch gekennzeichnet, dass** das Mengenverhältnis der Extrakte von Allium sativum, Juglans regia, Echinacea purpurea, Viola tricolor, Uncaria tomentosa und Carduus marianus ungefähr 3:2:3:4:3:2 beträgt.

7. Phytotherapeutische Zusammenstellung nach einem der vorangegangenen Ansprüche, **dadurch gekennzeichnet, dass** die Zusammenstellung darüber hinaus ein Extrakt von Eleutherococcus senticosus enthält.

8. Phytotherapeutische Zusammenstellung nach Anspruch 1, 3, 5 und 7, **dadurch gekennzeichnet, dass** das Mengenverhältnis der Extrakte von Allium sativum, Eleutherococcus senticosus, Juglans regia, Echinacea purpurea, Viola tricolor, Uncaria tomentosa en Carduus marianus ungefähr 3:3:2:3:4:3:2 beträgt.

9. Phytotherapeutische Zusammenstellung nach Anspruch 1, 3, 5 und 7 oder nach Anspruch 8, **dadurch gekennzeichnet, dass** der Volumenanteil des Allium-sativum-Extrakts an der Zusammenstellung 1 bis 30 Prozent, des Eleutherococcus-senticosus-Extrakts 1 bis 30 Prozent, des Juglans-regia-Extrakts 1 bis 25 Prozent, des Echinacea-purpurea-Extrakts 1 bis 30 Prozent, des Viola-tricolor-Extrakts 5 bis 35 Prozent, des Uncaria-tomentosa-Extrakts 1 bis 30 Prozent und des Carduus-marianus-Extrakts 1 bis 25 Prozent beträgt.

10. Phytotherapeutische Zusammenstellung nach Anspruch 9, **dadurch gekennzeichnet, dass** der Volumenanteil des Allium-sativum-Extrakts an der Zusammenstellung genau oder nahezu 15 Prozent, des Eleutherococcus-senticosus-Extrakts 15 Prozent, des Juglans-regia-Extrakts 10 Prozent, des Echinacea-purpurea-Extrakts 15 Prozent, des Viola-tricolor-Extrakts 20 Prozent, des Uncaria-tomentosa-Extrakts 15 Prozent und des Carduus-marianus-Extrakts 10 Prozent beträgt.

## Revendications

1. Composé phytothérapique contenant des extraits d'Allium sativum, de Juglans regia, d'Echinacea purpurea et de Viola tricolor.

2. Composé phytothérapique selon la revendication 1, **caractérisé en ce que** le rapport de quantité d'extraits d'Allium sativum, de Juglans regia, d'Echinacea purpurea et de Viola tricolor est d'environ 3:2:3:3.

3. Composé phytothérapique selon la revendication 1 ou 2, **caractérisé en ce que** le composé contient en outre un extrait de Carduus marianus.

4. Composé phytothérapique selon la revendication 3, **caractérisé en ce que** le rapport de quantité d'extraits d'Allium sativum, de Juglans regia, d'Echinacea purpurea, de Viola tricolor et de Carduus marianus est d'environ 3:2:3:3:2.

5. Composé phytothérapique selon une des revendications précédentes, **caractérisé en ce que** le composé contient en outre un extrait d'Uncaria tomentosa.

6. Composé phytothérapique selon les revendications 1, 3 et 5, **caractérisé en ce que** le rapport de quantité d'extraits d'Allium sativum, de Juglans regia, d'Echinacea purpurea, de Viola tricolor, d'Uncaria tomentosa et de Carduus marianus est d'environ 3:2:3:4:3:2.

7. Composé phytothérapique selon une des revendications précédentes, **caractérisé en ce que** le composé contient en outre un extrait d'Eleuterococcus scenticosus.

8. Composé phytothérapique selon les revendications 1, 3, 5 et 7, **caractérisé en ce que** le rapport de quantité d'extraits d'Allium sativum, d'Eleuterococcus scenticosus, de Juglans regia, d'Echinacea purpurea, de Viola tricolor, d'Uncaria tomentosa et de Carduus marianus est d'environ 3:3:2:3:4:3:2.

9. Composé phytothérapique selon les revendications 1, 3, 5 et 7 ou selon la revendication 8, **caractérisé en ce que** le composé contient, en pourcentage de volume, 1-30 % d'extrait d'Allium sativum, 1-30 % d'extrait d'Eleuterococcus scenticosus, 1-25 % d'extrait de Juglans regia, 1-30 % d'extrait d'Echinacea purpurea, 5-35 % d'extrait de Viola tricolor, 1-30 % d'extrait d'Uncaria tomentosa et 1-25 % d'extrait de Carduus marianus.

10. Composé phytothérapique selon la revendication 9, **caractérisé en ce que** le composé contient en tout cas pratiquement 15% en volume d'extrait d'Allium sativum, 15% en volume d'extrait d'Eleuterococcus scenticosus, 10% en volume d'extrait de Juglans regia, 15% en volume d'extrait d'Echinacea purpurea, 20% en volume d'extrait de Viola tricolor, 15% en volume d'extrait d'Uncaria tomentosa et 10% en volume d'extrait de Carduus marianus.
